# EUROPÄISCHE PATENTSCHRIFT

(11) **EP 0 372 362 B1**
(45) Veröffentlichungstag und Bekanntmachung des Hinweises auf die Patenterteilung: **28.06.1995**
(21) Anmeldenummer: 89121958.6
(22) Anmeldetag: 28.11.1989
(51) Int. Cl.: A61B 17/36

(54) **Laserskalpell**
Laser scalpel
Scalpel à laser

(30) Priorität: 02.12.1988 DE 3840609
(43) Veröffentlichungstag der Anmeldung: 13.06.1990
(73) Patentinhaber: KRISTALLTECHNOLOGIE ANDREAS MAIER GmbH, D-88477 Schwendi-Hörenhausen (DE)
(72) Erfinder: Ackermann, Lothar, Dr., D-7918 Illertissen (DE)
(74) Vertreter: König, Beate, Dipl.-Phys. Dr.

(56) Entgegenhaltungen:
- EP-A- 0 227 258
- EP-A- 0 292 622
- DE-A- 3 730 278

## Beschreibung

Die Erfindung bezieht sich auf ein Laserskalpell zur chirurgischen und dermatologischen Anwendung insbesondere am lebenden Menschen, mit einem Faseranschluß zur Zuführung der Laserenergie.

Für die Anwendung in der Laserchirurgie ist für die Arbeit mit Laserstrahlen ein geeignetes optisches Sytem erforderlich, um den Laserstrahl in der Praxis handhaben zu können und möglichst gute und reproduzierbare Eigenschaften in der Schneid-, Koagulations- und Vaporisationswirkung zu erreichen.

Dieses System soll bei Routineeingriffen am lebenden menschlichen Gewebe einsetzbar sein und einen möglichst störungsfreien Ablauf des Eingriffs gewährleisten.

Das Skalpell soll problemlos sterilisierbar und demontierbar sein, wobei die Montage der Skalpellspitzen ohne Bruchgefahr für die eigentlichen Spitzen erfolgen soll.

Für die ansich bekannte Übertragung des Laserstrahls vom Erzeugungssystem zur Skalpellspitze als Auskopplungsmedium sind verschiedene Applikationen der Auskopplung am Ende der Lichtleitfasern eingesetzt.

Z.B. ist eine konvexe Auskoppelfläche mit Fokusierung des Laserstrahles bekannt, mit Paralell- oder Streustrahlung. Dabei ist die Wirkung und damit die Schneidtiefe gering. Die Faserenden werden hoch beansprucht und haben eine relativ kurze Einsatzzeit. Außerdem besteht Bruchgefahr infolge Thermoschock bei Berührung mit Flüssigkeit. Diese Störungen führen häufig zur Unterbrechung des Eingriffs.

Darüber hinaus sind aus WO 85/05262 und WO 05623 Saphirspitzen bekannt, die im Einsatz direkt mit dem Gewebe in Kontakt gebracht werden; sie bewirken aufgrund der hohen Erwärmung eine zusätzliche thermische Schneid- und Koagulationswirkung.

Dieser Werkstoff erfordert eine Herstellung exakt in der optischen Achse des Kristalls um Polarisationseffekte zu vermeiden.

Geringe Abweichungen von dieser Achse bewirken eine Änderung der Brechungsindizes für den durchlaufenden Strahl und eine Linearpolarisation desselben, was zu einem undefinierten Strahlverlauf und damit zu einem daraus resultierenden nicht reproduzierbaren Strahlprofil führt.

Die Folge sind nicht reproduzierbare Schneid- und Koagulationsergebnisse.

Im klinischen Einsatz zeigte sich, daß die Schmelztemperatur des Saphirs häufig überschritten wird, was vermutlich infolge der Strahlabsorption an Einschlüssen erfolgt. Daneben wurden häufig explosionsartige Zerstörungen der Spitzen im Einsatz beobachtet.

Ein weiterer Nachteil des Saphiers ist die geringe mechanische Belastbarkeit im Winkel von etwa 60° zur optischen Achse.

Bekannt ist es darüber hinaus, Skalpellspitzen mit Ringnuten zu versehen, in die ein Teil der Aufnahmenhülse zur Sicherung gegen herausfallen eingerollt wird. Dabei besteht die Gefahr der Kerbwirkung in der Nut und die Gefahr, daß die Spitze im Gebrauch oder bei mechanischer Beanspruchung bricht.

Außerdem sind Sterilisierung und Reinigung wegen der engen Ringspalte problematisch.

Der Erfindung liegt die Aufgabe zugrunde, ein Laserskalpell so auszugestalten, daß die eine einwandfreie Benutzung gewährleistet ist.

Diese Aufgabe wird erfindungsgemäß dadurch gelöst, daß die Skalpellspitze aus monokristallinem Spinell im System MgO-Al₂O₃ besteht.

Die Vorteile liegen im höheren Schmelzpunkt des Werkstoffs, der eine höhere Arbeittemperatur ermöglicht. Da der Werkstoff optisch isotrop ist, muß bei der Bearbeitung eine Kristallachse nicht berücksichtigt werden.

Damit verbunden sind eine höhere Schneidleistung, kein Abschmelzen der Spitzen und eine höhere Unempfindlichkeit gegen Thermoschocks.

Bedingt durch die besseren Werkstoffwerte besteht die Möglichkeit, Skalpellspitzen mit Schneiden herzustellen, die es wahlweise ermöglichen, einen Eingriff mit oder ohne Laserunterstützung durchzuführen.

Als sehr vorteilhaft hat es sich dabei erwiesen, daß die Skalpellspitze herausnehmbar in einer Halterung gelagert ist.

Dadurch ist insbesondere die Reinigung der Skalpellspitze sehr einfach durchzuführen.

Sehr vorteilhaft ist es auch, wenn erfindungsgemäß eine koaxiale Kühlmittelzuführung vorgesehen ist, durch welche die die Laserenergie führende Faser mit Kühlmittel umspült wird.

Dabei ist erfindungsgemäß eine außerhalb der Kühlmittelzuführung angeordnete Kühlmittelrückführung vorgesehen.

Die Kühlflüssigkeit sorgt dabei sehr wirksam für eine ausreichende Kühlung des Endes der Lichtleitfaser und unterbindet die Bildung von Gasblasen. Dabei ist gewährleistet, daß keine Kühlflüssigkeit aus dem Ende des Skalpells austritt, wodurch der Blick auf die Eingriffstelle beeinträchtigt würde.

Sehr vorteilhaft ist es auch, daß erfindungsgemäß die Halterung für die Skalpellspitze mit mehreren nach innen gerichteten Führungs-Nocken für die Skalpellspitze ausgerüstet ist.

Dadurch wird nicht nur ein guter Halt für die Skalpellspitze erzielt, sondern auch der Kühlmittelfluß nicht beeinträchtigt.

Eine weitere vorteilhafte Ausgestaltung der Erfindung liegt darin, daß die Skalpellspitze mit einer messerartigen Schneide versehen ist.

Dadurch wird der Einsatz auch ohne Unterstützung mit Laserstrahlen besonders begünstigt.

Sehr vorteilhaft ist es auch, wenn erfindungsgemäß die Skalpellspitze mit einer Bohrung versehen ist, in welche der Faserleiter zur Zuführung der Laserenergie einführbar ist.

Damit ist ein besonders effektiver Übergang der Laserenergie vom Lichtleiter zur Laserspitze gewährleistet.

Dabei ist es besonders günstig und wirtschaftlich realisierbar, wenn erfindungsgemäß der Faserleiter in die Bohrung eingeklebt ist.

Es ist aber auch möglich, den Faserleiter gemäß einer weiteren Ausgestaltung der Erfindung in die Bohrung einzuschmelzen.

Eine weitere Möglichkeit für die Befestigung besteht darin, daß der Faserleiter in die Bohrung unter Zuhilfenahme eines entsprechenden Lotes eingelötet ist.

Eine weitere vorteilhafte Ausgestaltung der Erfindung liegt darin, daß die Außenfläche der Skalpellspitze mit einer reflektierenden Metallschicht versehen ist, die im Bereich des Anschlusses des Faserleiters und der Arbeitsfläche unterbrochen ist.

In der Zeichnung ist die Erfindung anhand mehrerer Ausführungsbeispiele veranschaulicht. Dabei zeigen:
- Fig.1 bis 5: verschiedene Ausgestaltungen von Skalpellspitzen,
- Fig.6: eine Skalpellspitze mit Bohrung, in welcher ein Lichtleiter eingelassen ist,
- Fig.7: einen Schitt durch das Endstück eines Laserskalpells mit Kühlmittelführung und
- Fig.8 bis 11: verschiedene Ausführungen der Spitzenlagerung im Handgriff des Laserskalpells.

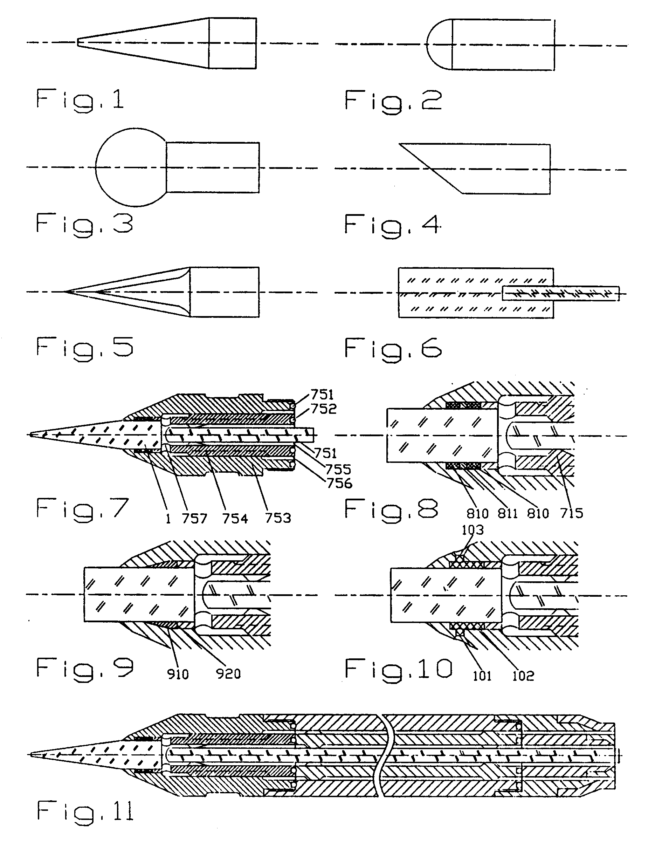

Wie in den Figuren 1 bis 5 dargestellt wurde, kann die Skalpellspitze für die verschiedenen Einsatzzwecke unterschiedlich ausgestaltete Formen aufweisen.

Am hinteren Ende ist die Skalpellspitze wie Fig.6 zeigt mit einer Bohrung versehen, in welche eine als Lichtleitfaser dienende Glasfaser eingesteckt ist. Zur Befestigung kann die Glasfaser eingeklebt, eingeschmolzen oder mit Glaslot eingelötet sein.

Wie besonders aus den Fig.7 und 8 ersichtlich ist, kann auch eine Halterung vorgesehen sein, die mit mehreren nach innen gerichteten Führungs-Nocken 715 versehen ist, zwischen denen die Lichtleitfaser gehalten wird. dadurch bilden sich zusätzlich Kanäle 755 aus, durch welche ein Kühlmittel für das Ende der Lichtleitfaser zugeführt werden kann.
Am der eigentlichen Skalpellspitze zugekehrten Ende der Halterung sind radiale Bohrungen 757 vorgesehen, welche in Rückflußkanäle 756 münden, wodurch ein geschlossener Kreislauf für das Kühlmittel geschaffen ist.

Zur Abdichtung des Spitzenhalters und des koaxialen Kühlsystems gegen den Skalpellgriff sind axial wirkende Dichtringe 751,752 vorgesehen, die eine zuverlässige Abdichtung gewährleisten und bei einer Demontage des Systems zur Überholung oder Sterilisation leicht ausgetauscht werden können.

Zur Fixierung der Skalpellspitze kann ein keilförmiger Ring 910(Fig.9) vorgesehen sein, mit dem die Spitze verspannt werden kann. Dieser Ring 910 ist vorzugsweise aus Silber hergestellt, wodurch ein guter Wärmeübergang gewährleistet ist.

Es ist aber auch möglich für die Fixierung der Spitze axial verspannte Dichtringe 810,811 vorzusehen, mit denen ein leichtes Auswechseln bzw. Demontieren der Spitzen gewährleistet ist.

Zur dauerhaften Festlegung der Spitze ist es möglich, einen Ringkanal mit keramischer Vergußmasse 101,102,103 vorzusehen.

## Patentansprüche

1. Laserskalpell zur chirurgischen und dermatologischen Anwendung insbesondere am lebenden Menschen, mit einem Faseranschluß zur Zuführung der Laserenergie, **dadurch gekennzeichnet**, daß die Skalpellspitze aus monokristallinem Spinell im System MgO-Al₂O₃ besteht.

2. Laserskalpell nach Anspruch 1, **dadurch gekennzeichnet**, daß die Skalpellspitze herausnehmbar in einer Halterung(753) gelagert ist.

3. Laserskalpell nach Anspruch 1 od. 2, **dadurch gekennzeichnet**, daß eine koaxiale Kühlmittelzuführung(755) vorgesehen ist, durch welche die die Laserenergie führende Faser mit Kühlmittel umspült wird.

4. Laserskalpell nach Anspruch 3, **dadurch gekennzeichnet**, daß eine außerhalb der Kühlmittelzuführung(755) angeordnete Kühlmittelrückführung(756) vorgesehen ist.

5. Laserskalpell nach Anspruch 3 od. 4, **dadurch gekennzeichnet**, daß die Halterung für die Skalpellspitze mit mehreren nach innen gerichteten Führungs-Nocken(715) für die Lichtleitfaser ausgerüstet ist.

6. Laserskalpell nach einem der vorhergehenden Ansprüche, **dadurch gekennzeichnet**, daß die Skalpellspitze mit einer messerartigen Schneide versehen ist.

7. Laserskalpell nach einem der vorhergehenden Ansprüche, **dadurch gekennzeichnet**, daß die Skalpellspitze mit einer Bohrung versehen ist, in welche der Faserleiter zur Zuführung der Laserenergie einführbar ist.

8. Laserskalpell nach Anspruch 7, **dadurch gekennzeichnet**, daß der Faserleiter in die Bohrung eingeklebt ist.

9. Laserskalpell nach Anspruch 7, **dadurch gekennzeichnet**, daß der Faserleiter in die Bohrung eingeschmolzen ist.

10. Laserskalpell nach Anspruch 7, **dadurch gekennzeichnet**, daß der Faserleiter in die Bohrung unter Zuhilfenahme eines entsprechenden Lotes eingelötet ist.

11. Laserskalpell nach einem der vorhergehenden Ansprüche, **dadurch gekennzeichnet**, daß die Außenfläche der Skalpellspitze mit einer reflektierenden Metallschicht versehen ist, die im Bereich des Anschlusses des Faserleiters und der Arbeitsfläche unterbrochen ist.

## Claims

1. Laser scalpel for surgical and dermatological use in particular on living people, with a fibre connection for the supply of laser energy, characterised in that the tip of the scalpel consists of monocrystalline spinel of the MgO-Al₂ O₃ system.

2. Laser scalpel according to Claim 1, characterised in that the scalpel tip is removably mounted in a mounting (753).

3. Laser scalpel according to Claim 1 or 2, characterised in that a coaxial coolant supply (755) is provided, due to which coolant flows around the fibres carrying the laser energy.

4. Laser scalpel according to Claim 3, characterised in that a coolant return (756) is provided, which is arranged outside the coolant supply (755).

5. Laser scalpel according to Claim 3 or 4, characterised in that the mounting for the scalpel tip is equipped with several inwardly directed guide cams (715) for the light-guide fibres.

6. Laser scalpel according to one of the preceding Claims, characterised in that the scalpel tip is provided with a knife-like cutting edge.

7. Laser scalpel according to one of the preceding Claims, characterised in that the scalpel tip is provided with a bore, into which the fibre conductor for supplying the laser energy can be introduced.

8. Laser scalpel according to Claim 7, characterised in that the fibre conductor is stuck in the bore.

9. Laser scalpel according to Claim 7, characterised in that the fibre conductor is fused in the bore.

10. Laser scalpel according to Claim 7, characterised in that the fibre conductor is soldered in the bore with the assistance of a corresponding solder.

11. Laser scalpel according to one of the preceding Claims, characterised in that the outer surface of the scalpel tip is provided with a reflecting metal layer, which is interrupted in the region of the connection of the fibre conductor and of the working surface.

## Revendications

1. Scalpel à laser destiné à être utilisé en chirurgie et en dermatologie, notamment sur l'être humain vivant, comportant un moyen de connexion de fibre pour l'introduction de l'énergie laser, caractérisé par le fait que la pointe du scalpel à laser est constituée par un spinelle monocristallin du système MgO-Al₂-O₃.

2. Scalpel à laser selon la revendication 1, caractérisé par le fait que la pointe du scalpel à laser est montée de manière démontable dans un support (753).

3. Scalpel à laser selon la revendication 1 ou la revendication 2, caractérisé par le fait qu'il est prévu une arrivée (755) coaxiale d'agent réfrigérant, grâce à laquelle la fibre qui achemine l'énergie laser est baignée par l'agent réfrigérant.

4. Scalpel à laser selon la revendication 3, caractérisé par le fait qu'il est prévu un retour (756) d'agent réfrigérant qui est disposé à l'extérieur de l'arrivée (755) d'agent réfrigérant.

5. Scalpel à laser selon la revendication 3 ou la revendication 4, caractérisé par le fait que le support pour la pointe de scalpel est pourvue de plusieurs saillies (715) tournées vers l'intérieur pour le guidage de la fibre optique.

6. Scalpel à laser selon l'une des revendications précédentes, caractérisé par le fait que la pointe de scalpel est pourvue d'une arête en forme de couteau.

7. Scalpel à laser selon l'une des revendications précédentes, caractérisé par le fait que la pointe de scalpel est pourvue d'un trou dans lequel la fibre optique peut être introduite aux fins d'amener l'énergie laser.

8. Scalpel à laser selon la revendication 7, caractérisé par le fait que la fibre optique est collée dans le trou.

9. Scalpel à laser selon la revendication 7, caractérisé par le fait que la fibre optique est scellée par fusion dans le trou.

10. Scalpel à laser selon la revendication 7, caractérisé par le fait que la fibre optique est soudée dans le trou au moyen d'un matériau de soudage adapté.

11. Scalpel à laser selon l'une des revendications précédentes, caractérisé par le fait que la surface extérieure de la pointe du scalpel est pourvue d'une couche métallique réfléchissante qui est in-terrompue dans la région de la connexion la fibre optique et de la surface de travail.
